# EUROPEAN PATENT APPLICATION

(11) **EP 1 354 580 A1**
(43) Date of publication of application: **22.10.2003**
(21) Application number: 01995033.6
(22) Date of filing: 28.12.2001
(51) Int. Cl.: A61K 7/48, A61K 7/00, A61K 45/00, A61K 31/047, A61K 31/185, A61K 31/205, A61K 31/375, A61K 31/785, A61K 35/78, A61K 38/06, A61P 17/16

(54) **AGENTS FOR INHIBITING OR RESTORING SKIN DAMAGE CAUSED BY DRYING AND METHOD OF EVALUATING THE SAME**

(30) Priority: 28.12.2000 JP 2000402005; 28.12.2000 JP 2000402085; 06.04.2001 JP 2001108422
(71) Applicant: SHISEIDO COMPANY LIMITED, Chuo-ku, Tokyo 104-8010 (JP)
(72) Inventor: KATAGIRI, Chika, c/o Shiseido Research Center, Yokohama-shi, Kanagawa 236-8643 (JP); HIRAO, Tetsuji, c/o Shiseido Research Center, Yokohama-shi, Kanagawa 2368643 (JP); FUJITA, Hiroshi, c/o Shiseido Research Center, Yokohama-shi, Kanagawa 236-8643 (JP); KOGA, Nobuyoshi, c/o Shiseido Research Center, Yokohama-shi, Kanagawa 224-8558 (JP)
(74) Representative: Albrecht, Thomas, Dr.
(86) International application number: JP0111615
(87) International publication number: WO02053127

(57) **Abstract**

External preparations for the skin which contain, as active ingredients, substances capable of preventing a reduction in the expression of the filaggrin gene in cultured human keratinocytes as induced by exposure to a gaseous phase (for example, betaines, polyols, crude drugs, and substances having an antioxidant action).

## Description

### Technical Field

This invention relates to cosmetics or dermatological preparations for inhibiting or restoring skin damage (or rough skin) caused by drying, and a method for evaluating substances contained in such preparations.

### Background Art

The natural moisturizing factors (NMF) perform an important function in maintaining the moisture content of the stratum corneum (Blank I.H., J. I. Dermatol., 18, 433(1952); Blank I.H., J. I. Dermatol., 21, 259(1953)). It has been reported that amino acids forming the principal constituents of NMF are produced by the proteoliticaly cleaved filaggrin originating from keratohyalin granules (Scott I.R. et al., Biochem. Biophys. Acta, 719, 110-117(1982) ; Horii I. et al., J. Dermatol., 10, 25-33(1983)). Filaggrin is a protein composed of 317 amino acids. Since it was clarified that amino acids forming the principal constituents of NMF are derived from filaggrin, investigations on the relation of morbid states exhibiting a dry skin to filaggrin have been carried forward. In recent years, it has been clarified that the amino acid content of the stratum corneum is reduced in a dry skin as seen in senile xerosis, atopic diseases and the like (Horii I. et al., Br. J. Dermatol., 121, 587-592(1989); Tanaka M. et al., Br. J. Dermatol., 139, 618-621(1998), and that the expression of filaggrin in such a dry skin is decreased (Tezuka T. et al., Dermatology, 188, 21-24(1994); Seguchi T. et al., Arch. Dermatol. Res., 288, 442-446(1996)). Moreover, it is well known that skin troubles such as rough skin are caused by a dry environment.

Possibly from this background, FR2 777 185-A1 has proposed a composition for the treatment of wrinkles in which a substance stimulating the synthesis of filaggrin is combined with other active substances so as to act favorably on the regulation of moisture-retaining capacity. However, no description is given of what is specifically the substance stimulating the synthesis of filaggrin. Moreover, it is described in Japanese Patent Laid-Open No. 2000-26272 that a protein decomposition product derived from a powder of the seeds of white lupine (or Lupinus albus) has the effect of increasing mRNA for filaggrin in human keratinocytes and increasing the thickness of the stratum corneum. Furthermore, it is described in WO99/47117 that a composition containing a vitamin B3 compound acts as a humectant to increase the level of a protein selected from filaggrin, keratin and involucrin and to enhance the moisture-adsorbing or absorbing ability of the stratum corneum.

### Disclosure of the Invention

As described above, there have conventionally been proposed substances which promote the production of filaggrin and increase the thickness of the stratum corneum or which enhance the moisture-absorbing capacity of the stratum corneum. However, it would still be desirable to provide substances or compounds which are recognized to be effective from a wider point of view, i.e., which participate in the production of filaggrin and can maintain a healthy skin condition.

As a result of investigations on factors associated with drying and the moisture content of the stratum corneum in human epidermides, the present inventors have found that the protein level of filaggrin and the expression of its gene are reduced by drying.

Moreover, it has been found that, when a certain type of cultured human keratinocytes are exposed to a gaseous phase, the expression level of the filaggrin gene is significantly reduced and this phenomenon is correlated with the aforesaid skin damage caused by drying. It has also been found that, in a culture system using the cultured human keratinocytes, the aforesaid skin damage caused by drying can be can be inhibited or restored by a substance capable of inhibiting significantly a reduction in the expression level of the filaggrin gene as induced by exposure to a gaseous phase.

The present invention is based on these findings.

Accordingly, the present invention provides an external preparation for the skin comprising a substance capable of inhibiting significantly a reduction in the expression level of the filaggrin gene in cultured human keratinocytes as induced by exposure to a gaseous phase, in a sufficient amount to inhibit a reduction in the barrier function of the stratum corneum in the human skin; and a base or additive acceptable for use in cosmetics and/or dermatological preparations.

In a preferred embodiment of the present invention, there is provided an external preparation for the skin wherein the aforesaid substance capable of inhibiting significantly a reduction in the expression level of the filaggrin gene comprises one or more members selected from the group consisting of betaine compounds and derivatives thereof, polyols and derivatives thereof, crude drugs, and substances having an antioxidant action.

The present invention also relates to the use of a substance capable of inhibiting significantly a reduction in the expression level of the filaggrin gene in cultured human keratinocytes as induced by exposure to a gaseous phase, for the making of an external preparation for the skin which can inhibit a reduction in the barrier function of the stratum corneum in the human skin and the moisture-retaining function thereof.

Moreover, the present invention relates to a method for inhibiting or restoring skin damage caused by drying which comprises the step of applying to the human skin a composition containing one or more members selected from the group consisting of betaine compounds and derivatives thereof, polyols and derivatives thereof, crude drugs, and substances having an antioxidant action.

In another embodiment of the present invention, there is provided a method for evaluating whether a substance can inhibit skin damage caused by drying or not. This evaluation method comprises
(A) providing cultured human keratinocytes,
(B) after the cultured human keratinocytes are exposed to a gaseous phase in the presence of a test substance, detecting the expression level of the filaggrin gene in the keratinocytes, and
(C) comparing the detected expression level of the filaggrin gene with that of a control, and regarding the level of the expression as an index to the skin damage-inhibiting effect.

### Brief Description of the Drawings

FIG. 1 is a graph showing relative changes in filaggrin protein level with time as caused by drying (exposure to a gaseous phase) of cultured human keratinocytes. The data are expressed by the mean ±SEM value in a number of trials (n = 4-5). ^{**} means p<0.01.
FIG. 2 is a graph showing changes in the expression level of the filaggrin gene with time as caused by drying (exposure to a gaseous phase) of cultured human keratinocytes. The data are expressed by the mean ±SEM value in a number of trials (n = 4-6). ^{**} means p<0.01, and ^{*} means p<0.05.
FIG. 3 is a graph showing the effect of glycerol inhibiting a reduction in filaggrin protein level as induced by drying (exposure to a gaseous phase) of cultured human keratinocytes. In each pair of bars, the right-hand one shows an example of culture in the presence of 10% glycerol (the left-hand one is a control). The data are expressed by the mean ±SEM value in a number of trials (n = 3-6). ^{**} means p<0.01, and ^{*} means p<0.05.
FIG. 4 is a graph showing the effect of glycerol and trimethylglycine on a reduction in the expression of the filaggrin gene as induced by drying of cultured human keratinocytes. The data are expressed by the mean ±SEM value in a number of trials (n = 3). ^{**} means p<0.01.
FIG. 5 is a graph having the same meaning as FIG. 4, except that crude drugs were used as drugs. The concentration of each drug is 0.0025% on a dry residue basis. n = 3, ^{*} means p<0.05, ^{**} means p<0.01, and ^{***} means p<0.001.
FIG. 6 is a graph having the same meaning as FIG. 4, except that ascorbic acid and glutathione were used as drugs at a concentration of 1 mM. ^{***} means p<0.001, and ^{**} means p<0.01.
FIG. 7 is a graph having the same meaning as FIG. 4, except that hypotaurine was used as a drug. ^{*} means p<0.05.

### Best Mode for Carrying Out the Invention

As used herein, the expression "capable of inhibiting significantly a reduction in the expression level of the filaggrin gene in cultured human keratinocytes as induced by exposure to a gaseous phase" means that, when evaluated according to an evaluation method which will be described later, a reduction in the aforesaid expression level of filaggrin in a system containing a test substance can be inhibited to a statistical significant degree, as compared with a reduction in the aforesaid expression level of filaggrin in a control system (containing no test substance).

The term "skin damage" as used herein means various kinds of changes or skin troubles which are not observed in a healthy skin, such as a rough skin, the formation of fine wrinkles in the skin, and a reduction in the softness of the skin.

In the present invention, any substances effect of inhibiting significantly a reduction in the aforesaid expression level fall under the category of active ingredients for use in the external preparation, use and method of the present invention. However, specific examples-of such active ingredients include betaine compounds and derivatives thereof, polyols and derivatives thereof, crude drugs, and substances having an antioxidant action.

The "betaine compounds and derivatives thereof' may be any compounds included in betaine (also called glycinebetaine) and its derivatives, and zwitter ion compounds of the corresponding sultaine (in which the carboxyl of betaine is replaced by sulfo), provided that they meet the purposes of the present invention. Representative examples of such derivatives include compounds formed by replacing one to three of the N-methyl groups of glycinebetaine with other optionally branched saturated or unsaturated hydrocarbon chain(s) or such hydrocarbon chain(s) interrupted by an amide linkage of the formula (CₙH₂ₙCONH(CH₂)ₘ⁻) wherein m and n are independently integers of 1 to 30; compounds formed by varying the length of the hydrocarbon chain between the quaternary ammonium group and the carboxyl or sulfo group (for example, from 2 to 6 carbon atoms); and polymers having a plurality of betaine residues as pendant groups. Moreover, they may be cyclic compounds. In addition to the aforesaid compounds, specific examples of such derivatives include, but are not limited to, γ-butyrobetaine, decylbetaine, laurylbetaine, myristylbetaine, cetylbetaine, stearylbetaine, behenylbetaine, lauramidopropylbetaine, oleamidopropylbetaine, palmitamidopropylbetaine, γ-butyrobetaine, laurylsultaine, coco-sultaine, poly(methacryloyloxyethylbetaine), poly(methacryloyloxyethylbetaine-co-2-hydroxyethylmethacrylic acid) and the like. Preferred derivatives are glycinebetaine, sultaine, poly(methacryloyloxyethylbetaine) and poly(methacryloyloxyethylbetaine-co-2-hydroxyethylmethacrylic acid).

The "polyols and derivatives thereof" include compounds having two or more hydroxyl groups per molecule, and derivatives thereof, such as ethylene oxide adducts. Specific examples of such polyols include glycerol, 1,3-propanediol, 2-methyl-1,3-propanediol, 1,4-butanediol, 1,5-pentanediol, diglycerol, erythritol, gluconic acid, 1,2,6-hexanetriol, inositol, lactitol, maltitol, mannitol, xylitol and the like. In particular, it is preferably to use a combination of glycerol and a sugar alcohol such as xylitol.

The "crude drus" are medicines or folk medicines, or raw materials therefore, which have been obtained by processing materials of vegetable origin according to such techniques as drying, cutting, grinding and extraction. It does not matter what family or genus such plants belong to, provided that they meet the purposes of the present invention. Specific examples of the crude drugs include, but are not limited to, crude drug carqueja (South America) derived from a plant of the genus Baccharis, particularly Baccharis genistelloides, crude drug macelamista (South America) derived from a plant of the genus Achyrocline. particularly Achyrocline satureoides, and crude drug achillea millefolium derived from a plant of the genus Achillea, particularly Achillea millefolium L., these genera belonging to the family Compositae ; crude drug thymus serphyllum extract derived from a plant of the genus Thymus. particularly Thymus serphyllum Linne, subsp. serphyllum, crude drug majoram extract derived from a plant of the genus Origanum, particularly Origanum majorana L., crude drug scuttellaria root derived from a plant of the genus Scuttellaria, particularly Scuttellaria baicalensis Georgi, and crude drug lavender oil derived from a plant of the genus Lavandula, particularly Lavandula officinalls. these genera belonging to the family Labiatae; crude drug rosa roxburghii fruit derived from a plant of the genus Rosa, particularly Rosa roxburghii, and crude drug cherry leaf extract derived from a plant of the genus Prunus, particularly Prunus lannesiana (Corr.) Wilson var. Spciosa (Koidz) Makino, these genera belonging to the family Rosaceae; crude drug hamamelis derived from a plant of the genus Hamamelis of the family Hamamelidaceae, particularly Hamamelis virginiana L.; crude drug pyrola derived from a plant of the genus Pyrola of the family Pyrolaceae. particularly Pyrola japonicus Klenze; and crude drug palo azul (South America) derived from a plant of the genus Eysenhardtia of the family Leguminosae. particularly Eysenhardtia polistachva. Preferred crude drugs include, but are not limited to, crude drug palo azul, crude drug macelamista, and crude drug carqueja.

The "substances having an antioxidant action" may be any compounds or substances (mixtures, compositions or the like) that can achieve the purposes of the present invention without exerting an adverse influence on the skin. From the viewpoint of exerting no adverse influence on the skin, specific examples thereof include compounds or substances which have already been used as antioxidants or have been proposed for use, for example, in the field of cosmetics.

Such compounds include, but are not limited to, ascorbic acid and salts or derivatives thereof. The salts or derivatives include, for example, magnesium ascorbate, sodium ascorbate and the like; and ascorbic acid derivatives such as ascorbic acid alkyl esters, ascorbic acid phosphoric esters, ascorbic acid sulfuric esters and ascorbic acid glucosides. More specifically, ascorbic acid alkyl esters include ascorbyl palmitate, ascorbyl isopalmitate, ascorbyl dipalmitate, ascorbyl diisopalmitate, ascorbyl stearate, ascorbyl isostearate, ascorbyl distearate, ascorbyl myristate, ascorbyl isomyristate, ascorbyl dimyristate, ascorbyl diisomyristate, ascorbyl 2-ethylhexanoate, ascorbyl di(2-ethylhexanoate), ascorbyl oleate, ascorbyl dioleate and the like; and other derivatives include ascorbic acid polypeptide, magnesium ascorbyl phosphate, methylsilanol ascorbate, potassium ascorbyl tocopheryl phosphate and the like.

Moreover, such compounds include tocopherol and derivatives thereof. The derivatives include, but are not limited to, tocopherol esters such as tocopheryl acetate, tocopheryl linoleate, tocopheryl nicotinate and tocopheryl succinate; and tocopherol-ethylene oxide adducts such as tocophereth-5, tocophereth-10 and tocophereth-12. Furthermore, such compounds include sulfur-containing amino acid containing compounds (including sulfur-containing amino acids themselves) and intermediary metabolites thereof, such as acetylcysteine, methionine, cysteine, homocysteine, glutathione, hypotaurine, cysteinesulfinic acid, cysteic acid, thiocysteine, taurine, djenkolic acid, cystathionine, S-allylcycteine, lenthionine and ethionine.

Among them, hypotaurine is contained in main organs (e.g., heart, brain, liver and the like) of many mammals including human beings. Moreover, since its side effects on the skin, such as irritation, itching and rash) have not been reported. Hypotaurine is an aminoethyl compound having very high safety. Hypotaurine may be prepared from an available thio compound (e.g., cysteine) according to a conventionally known method, or a commercially available product may be used. Accordingly, the present inventors firmly contemplate the use of hypotaurine.

According to the present invention, the aforesaid active ingredient may comprise a combination of any of the above-described preferred crude drugs and other active ingredients. More preferably, there may be used a combination of palo azul as a crude drug, and either of glycerol and a sugar alcohol having 4 to 6 carbon atoms; palo azul as a crude drug, glycerol, and glutathione or hypotaurine; crude drug palo azul as a crude drug, glycerol and ascorbic acid; or crude drug palo azul as a crude drug, glycerol, and a betaine compound or its derivative. On the other hand, when active ingredients are used in combination, it is a preferred embodiment to combine either of glycerol and a sugar alcohol having 4 to 6 carbon atoms, with any of the crude drugs specifically enumerated above, particularly crude drug carqueja (South America), crude drug macelamista (South America) and crude drug palo azul (South America).

The above-described preparations may be made according to per se known methods, for example, those described in "Hirokawa Yakuyo Shokubutsu Daijiten", ed. by Masao Kijima et al., 1980, K.K. Hirokawa Shoten.

In addition to the aforesaid active ingredient(s), the external preparation of the present invention may further contain a base or additives, or other active ingredients, which are commonly used in cosmetics and/or dermatological preparations.

In the external preparation of the present invention, an active ingredient comprising a betaine compound, a polyol or a substance having an antioxidant action may be present in an amount of 0.0001 to 20% by weight, preferably 0.01 to 1.0% by weight, based on the total weight of the external preparation. The amount of a crude drug cannot be specified because its active ingredient content varies according to its dosage form. However, a crude drug may be present in an amount of 0.0001 to 20% by weight (about 5 × 10⁻⁷ to 2 × 10^{-1%} by weight on a dry basis), preferably 0.01 to 1.0% by weight (about 5 × 10⁻⁵ to 5 × 10^{-3%} by weight on a dry basis), based on the total weight of the external preparation. In any case, with consideration for the results obtained by testing their specific usage forms according to a testing method or evaluation method which will be described later, and for the results obtained by their trial use in volunteers or the like, those skilled in the field of dermatology can easily determine the optimum doses at which the aforesaid active ingredients exhibit their efficacy.

The external preparations of the present invention include cosmetics, drugs and quasi drugs which are applied to the skin. Accordingly, their dosage forms may vary widely, including an aqueous solution system, a solubilized system, an emulsified system, a powder system, a gel system, an ointment system, a cream, a water-oil two-layer system, a water-oil-powder three-layer system and the like. That is, for basal cosmetics, they may be widely used in the aforesaid various dosage forms, as facial cleansers, toilet water, milky lotions, creams, gels, essences (beauty lotions), packs, masks and the like. For makeup cosmetics, they may be widely used in the form of foundations and the like. Moreover, for drugs and quasi drugs, they may be widely used in the form of various ointments. The forms which can be assumed by the external preparation of the present invention are not limited to the above-described dosage forms and usage forms.

The aforesaid base ingredients may be selected according to these dosage forms and usage forms. Usable base ingredients or additives include water, lower alkanols, fatty oils, solid fats, waxes, ester oils, hydrocarbon oils, silicone resins, silicones, anionic surfactants, cationic surfactants, amphoteric surfactants, nonionic surfactants, lower alcohols, sterols, water-soluble polymers, sequestering agents, neutralizing agents, pH regulators, antimicrobial agents, perfumes, colorants and the like.

The efficacy of the above-described external preparations can be confirmed by applying them directly to volunteers. In advance of this step, however, their efficacy can also be confirmed according to a method for evaluating whether a substance can inhibit skin damage caused by drying or not, which is another embodiment of the present invention.

The keratinocytes used in this evaluation method may be any type of cells, provided that, when they are cultured in the presence or absence of a test substance, the variation in the amount of filaggrin protein and the expression level of the gene can be significantly detected. In a preferred embodiment, however, a cultured cell system consisting of normal cells of human preputial origin and a feeder layer formed of 3T3 cells of mouse origin (ATCC CRL-1658) may conveniently be used. This cell culture system may be produced, for example, according to the method described in Rheinwald et al., Cell, 6, 331-334(1975). Specifically, 3T3 cells are cultured in a suitable Petri dish, and treated with mitomycin C to form a feeder layer. Thereafter, normal human keratinocytes are inoculated onto the layer and cultured at 37°C, for example, in a 95% air-5% carbon dioxide environment, until they become confluent.

Usually, a cell culture system in the absence of a test substance (a control system) is preferably treated in parallel with a cell culture system in the presence of a test substance (a test system). However, it is also possible to culture human keratinocytes, in advance, under various conditions in the absence of a test substance, obtain values for the amount of filaggrin protein and the expression level of the filaggrin gene after exposure to a gaseous phase, and use these values as controls.

Preferably, a test substance is added to the test system after the cultured human keratinocytes have come to be confluent, and the incubation is continued for a short period of time (for example, 30 minutes).

The exposure of the cultured human keratinocytes to a gaseous phase may preferably be carried out by removing the culture medium and incubating them at 37°C, for example, in a 95% air-5% carbon dioxide environment. This exposure to a gaseous phase may usually be completed in a time of up to about 6 hours.

After the cultured human keratinocytes are exposed to a gaseous phase, the amount of filaggrin protein or the expression level of the filaggrin gene is measured according to a per se known method. Thus, the degree of damage caused to human keratinocytes by exposure to a gaseous phase or the degree of the efficacy of a test substance against such damage can be evaluated. This evaluation method is also useful for the screening of additional active substances which can present skin damage associated with an in vivo decrease of filaggrin in human keratinocytes as induced by drying.

The present invention is further illustrated by the following specific examples. However, these examples are not to be construed to limit the scope of the invention. Unless otherwise stated, the percentages used therein are by weight.

### Tests for Measuring the Expression Level of the Filaggrin Gene:

### (1) Cell culture

Normal keratinocytes of human preputial origin and 3T3 cells of mouse origin were used.

### (2) Culture medium for cultured cells

The culture medium was DMEM-Ham's F12 (3:1) containing hydrocortisone, cholera enterotoxin, epidermal growth factor, insulin and 10% FBS.

### (3) Culture

The keratinocytes were cultured according to the method described in Rheinwald et al., Cell, 6, 331-344(1975).

An outline is given below. 3T3 cells were treated with mitomycin C and used as a feeder layer. Normal human keratinocytes (1 × 10⁵ cells) were inoculated onto mitomycin C-treated 3T3 cells and cultured at 37°C in a 95% air-5% carbon dioxide environment until they became confluent. They were exposed to a gaseous phase by removing the culture supernatant. Each test substance was added 30 minutes before exposed to a gaseous phase, followed by incubation at 37°C. The culture to which no test sample was added was used as a control. The exposure to a gaseous phase was carried out at 37°C for 0-6 hours.

### (4) Measurement of the amount of filaggrin protein

After each test substance was added to the aforesaid culture system and the cells were exposed to a gaseous phase for a predetermined period of time, protein was extracted from the cells. According to a conventional method, filaggrin protein or filaggrin protein precursor (profilaggrin) was determined by western blotting. An outline is given below. The extracted protein was electrophoresed in SDS-PAGE, transferred to a PDVF film, and subjected to an antigen-antibody reaction using a mouse monoclonal antibody specific for filaggrin (anti-HUMAN Filaggrin MAb) as a primary antibody. Thereafter, an antigen-antibody reaction was carried out by using a horseradish peroxidase- or alkaline phosphatase-labeled antibody as a secondary antibody. After the reaction, measurement was made according to a coloring method based on an enzyme reaction using a chromogenic substrate corresponding to the labeling enzyme, or a chemical coloring method. The quantitative analysis of bands was carried out with NIH Image (analytical program).

### (5) Measurement of the expression level of the filaggrin gene

After each test substance was added to the aforesaid culture system and the cells were exposed to a gaseous phase for a predetermined period of time, RNA was extracted from the cells according to the AGPC method. This RNA was subjected to RT-PCR in the following manner. A primer specific for filaggrin was prepared on the basis of the base sequence of filaggrin which is reported in McKinley-Grant L.J. et al., Proc. Natl. Acad. Sci. U.S.A., 86, 4848-4852(1989). In order to assay mRNA for filaggrin, RT-PCR was carried out by converting mRNA to cDNA with the aid of reverse transcriptase. Using this cDNA as a template with the aforesaid primer, a polymerase reaction (a well-known reaction) was carried out. After the PCR product was electrophoresed in agarose gel, the resulting gel was soaked in an ethidium bromide solution and assayed for DNA. The quantitative analysis of DNA bands was carried out with NIH Image (analytical program). A correction was made with the aid of glyceraldehyde-3-phosphate dehydrogenase (G3PDH).

### (6) Results

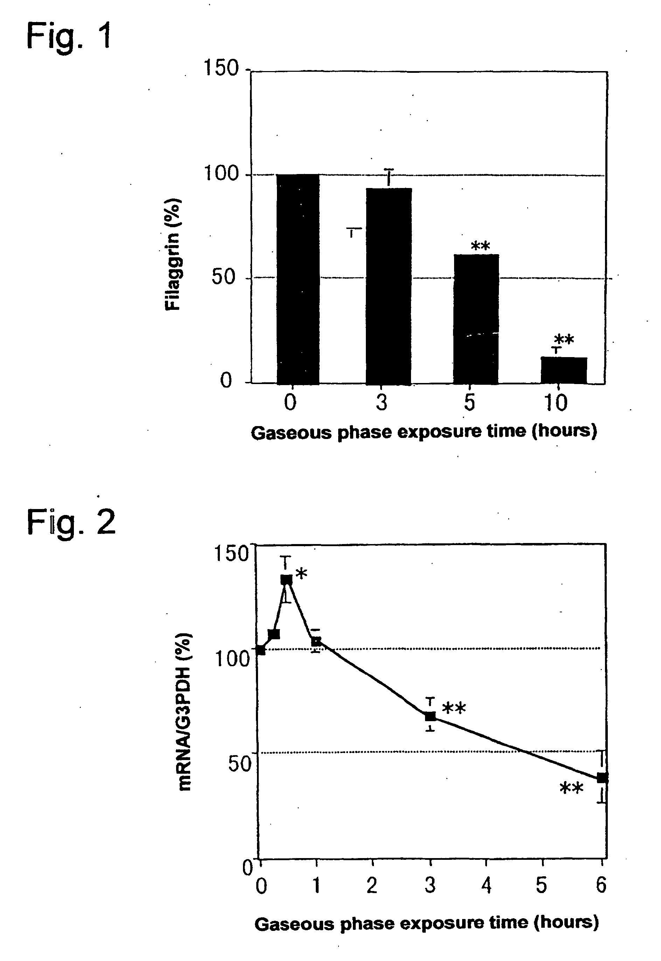

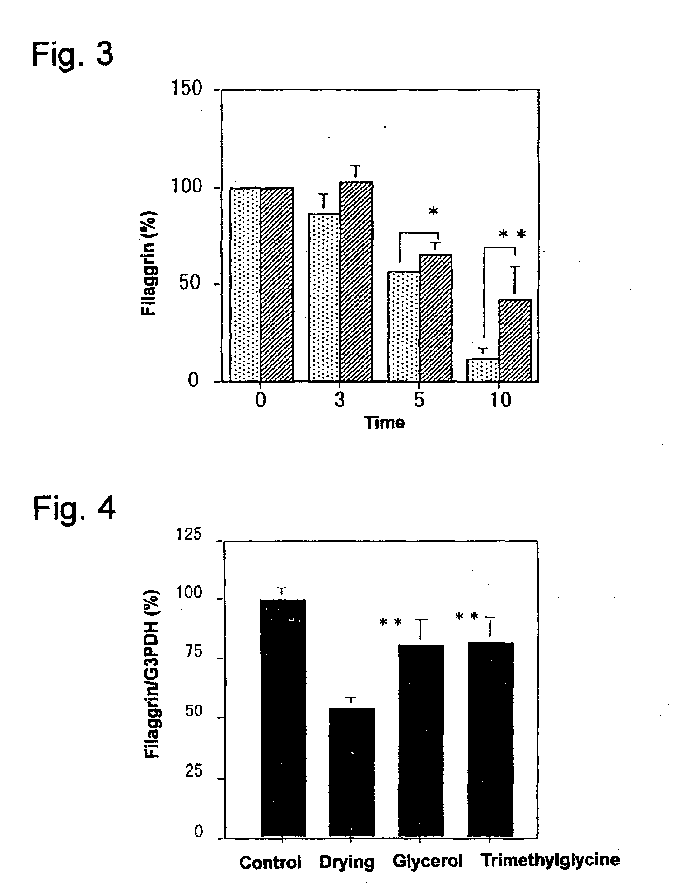

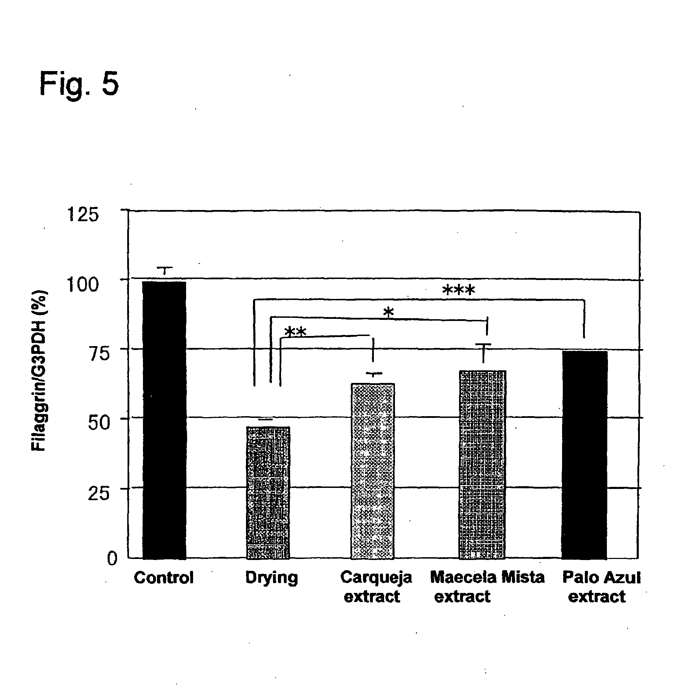

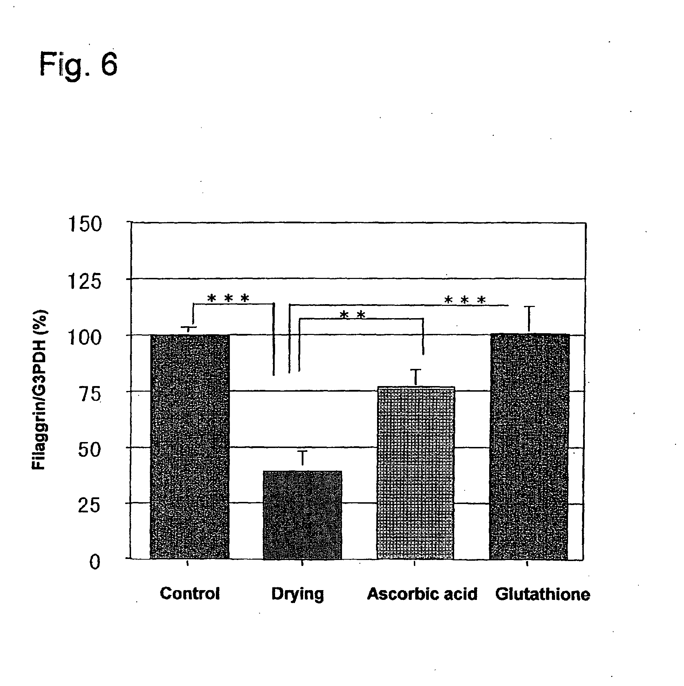

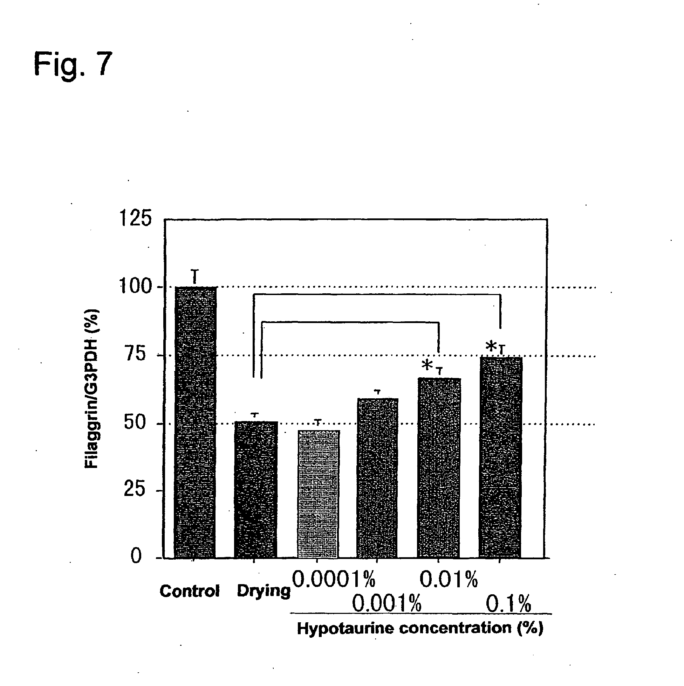

(6-1) Changes in the amount of filaggrin protein with time as caused by exposing human keratinocytes to a gaseous phase are shown in FIG. 1. It is evident from this figure that filaggrin protein in human keratinocytes is significantly decreased by drying.
(6-2) Changes in the expression level of the filaggrin gene in human keratinocytes with time as caused by the aforesaid exposure to a gaseous phase are shown in FIG. 2. It is evident from this figure that the expression of the filaggrin gene in human keratinocytes is significantly reduced by drying. Moreover, this reduction occurs in a relatively short time.
(6-3) The effect of inhibiting damage characterized by a reduction in the amount of filaggrin protein in human keratinocytes as induced by exposure to a gaseous phase (a drying stimulus) was evaluated in terms of the amount of filaggrin protein in the presence or absence of a sample (10% glycerol) added to the culture system. The results are shown in FIG. 3.
(6-4) The effect of inhibiting damage characterized by a reduction in the expression of the filaggrin gene in human keratinocytes as induced by exposure to a gaseous phase (a drying stimulus) was evaluated in terms of the expression level of the filaggrin gene in the presence or absence of a sample (glycerol or trimethylglycine) added to the culture system. The results are shown in FIG. 4.
(6-5) The effect of inhibiting damage characterized by a reduction in the expression of the filaggrin gene in human keratinocytes as induced by drying was evaluated in terms of the expression level of the filaggrin gene in the presence or absence of a crude drug extract added to the culture system. The results are shown in FIG. 5.
(6-6) The effect of inhibiting damage characterized by a reduction in the expression of the filaggrin gene in human keratinocytes as induced by drying was evaluated in terms of the expression level of the filaggrin gene in the presence or absence of ascorbic acid or glutathione added to the culture system. The results are shown in FIG. 6.
(6-7) The effect of inhibiting damage characterized by a reduction in the expression of the filaggrin gene in human keratinocytes as induced by drying is shown in FIG.7 in terms of the expression level of the filaggrin gene in the presence or absence of hypotaurine added to the culture system. Its concentration dependence is also shown therein.
(6-8) The efficacies of other active ingredients and combinations of active ingredients in inhibiting a reduction in the expression of the filaggrin gene in cultured human keratinocytes as induced by drying are shown in Tables 1 and 2 below, respectively.
In these tables, the degree to which the addition of a drug inhibited a reduction in gene expression is expressed as a percentage, assuming that the gene expression before drying is 100% and the gene expression after drying is 100%. In Table 1, each active ingredient was added at an extract concentration of 0.5%. In Table 2, compositions containing the indicated active ingredient(s) at the indicated concentration(s) (in % by weight) were tested.

**Table 1**

| Crude drug | Recovery rate (%) |
|---|---|
| Achillea millefolium | 41.9 |
| Majoram | 30.8 |
| Thymus serphyllum extract | 19.9 |
| Pyrola | 13.0 |
| Lavender | 8.6 |
| Hamamelis | 37.4 |
| Scutellaria liquid | 65.0 |
| Cherry leaf | 30.8 |
| Rosa roxburghii fruit | 53.1 |

**Table 2**

| Test substance | Relative expression level of filaggrin gene (%) | | |
|---|---|---|---|
| | Mean Mean | Standard error | Recovery rate (%) |
| No treatment | 100 | 6 | |
| Drying stimulus alone | 47 | 4 | |
| 0.025% palo azul extract | 60 | 6 | 24.5 |
| 0.15% glycerol | 51 | 13 | 7.5 |
| 0.30% glycerol | 79 | 7 | 60.4 |
| 0.15% xylitol | 50 | 23 | 5.7 |
| 0.30% xylitol | 67 | 16 | 37.7 |
| 0.15% glycerol + 0.15% xylitol | 78 | 14 | 58.5 |
| 0.025% palo azul extract + 0.15% glycerol + 0.15% xylitol | 94 | 5 | 88.7 |

The aforesaid recovery rates (%) indicate the degree to which the addition of each test substance restored the expression level of the filaggrin gene as compared with that in the untreated group not exposed to a drying stimulus.

From these figures and tables, it is confirmed that the preparations in accordance with the present invention can significantly inhibit a reduction in the moisture content of the stratum corneum in human keratinocytes as induced by drying, as well as a reduction in the amount of filaggrin protein, serving as a source of amino acids essential for the maintenance of moisture in the stratum corneum, and a reduction in the expression of the filaggrin gene.

Exemplary formulations for preparations in accordance with the present invention are described below. All of these preparations may be prepared in the usual manner. The contents of various ingredients in these preparations are expressed in percent by weight, unless otherwise specified.

| Formulation 1 (cream) | |
|---|---|
| Behenyl alcohol | 1.0 |
| Stearyl alcohol | 2.0 |
| Squalane | 10.0 |
| Pentaerythrityl tetraoctanoate | 5.0 |
| Lanolin | 5.0 |
| Paraben | 0.3 |
| Polyoxyethylene behenyl alcohol | 1.0 |
| Glycerol | 10.0 |
| Crude drug extract (crude drug carqueja) | 0.2 |
| Sodium acetylhyaluronate | 0.1 |
| Purified water | Balance |

| Formulation 2 (cream) | |
|---|---|
| Squalane | 10.0 |
| Isopropyl myristate | 20.0 |
| Decamethylcyclopentanehexane | 35.0 |
| Alkyl-modified carboxyvinyl polymer | 0.05 |
| Sorbitan monooleate | 5.0 |
| Dipropylene glycol | 3.0 |
| Crude drug extract (crude drug macelamista) | 0.2 |
| Purified water | Balance |

| Formulation 3 (cream) | |
|---|---|
| Liquid paraffin | 10.0 |
| Octamethylcyclotetrasiloxane | 5.0 |
| 1,3-Butyl glycol | 10.0 |
| Glycerol | 3.0 |
| Ascorbic acid derivative | 3.0 |
| Trimethylglycine | 0.5 |
| Carboxyvinyl polymer | 0.15 |
| Xanthane gum | 0.25 |
| Stearic acid | 1.5 |
| Cetyl alcohol | 0.5 |
| Polyoxyethylene(10) monooleate | 1.0 |
| Stearic acid monoglyceride | 1.0 |
| Ethanol | 3.0 |
| Perfume | q.s. |
| Purified water | Balance |

| Formulation 4 (cream) | |
|---|---|
| Cetostearyl alcohol | 3.5 |
| Squalane | 20.0 |
| Beeswax | 3.0 |
| Lanolin | 5.0 |
| Paraben | 0.3 |
| Polyoxyethylene(20) sorbitan monopalmitate | 2.0 |
| Stearic acid monoglyceride | 2.0 |
| Plant extract (crude drug palo azul) | 2.0 |
| Vitamin A | 2.0 |
| Perfume | q.s. |
| Purified water | Balance |

| Formulation 5 (cream) | |
|---|---|
| Talc | 3.0 |
| Titanium dioxide | 5.0 |
| Red iron oxide | 0.5 |
| Yellow iron oxide | 1.4 |
| Black iron oxide | 1.0 |
| Polyoxyethylene sorbitan monostearate | 0.9 |
| Triethanolamine | 1.0 |
| Propylene glycol | 5.0 |
| Crude drug extract (or trimethylglycine or neotrehalose) | 1.0 |
| Stearic acid | 2.2 |
| Isohexadecyl alcohol | 7.0 |
| Stearic acid monoglyceride | 2.0 |
| Lanolin | 2.0 |
| Paraffin | 8.0 |
| Paraben | q.s. |
| Perfume | q.s. |

| Formulation 6 (cream) | |
|---|---|
| Liquid paraffin | 6.0 |
| Vaseline | 2.0 |
| Methylpolysiloxane | 1.0 |
| Stearyl alcohol | 2.0 |
| Behenyl alcohol | 2.0 |
| Hardened oil | 1.0 |
| Squalane | 5.0 |
| Cetyl 2-ethylhexanoate | 4.0 |
| Self-emulsifiable glycerol monostearate | 2.0 |
| 4-tert-4'-Methoxybenzoylmethane | 0.1 |
| Macadamia nut oil | 1.0 |
| Sodium metaphosphate | 0.02 |
| Placenta extract | 1.0 |
| Paraben | q.s. |
| Colorant | q.s. |
| Glycerol | 5.0 |
| Dipropylene glycol | 5.0 |
| 1,3-Butylene glycol | 3.0 |
| Plant extract (crude drug palo azul) | 1.0 |
| Plant extract (plant rosa roxburghii fruit) | 0.5 |
| Purified water | Balance |

| Formulation 7 (cream) | |
|---|---|
| Methylpolysiloxane | 2.0 |
| Decamethylcyclopentasiloxane | 10.0 |
| Polyoxyethylene-methylpolysiloxane polymer | 3.0 |
| Dimethyldistearylammonium hectorite | 3.0 |
| Specially denatured alcohol | 2.0 |
| Glycerol | 5.0 |
| Dipropylene glycol | 3.0 |
| Polyethylene glycol | 1.0 |
| Sodium metaphosphate | 0.05 |
| Plant extract (crude drug palo azul) | 2.0 |
| Plant extract (crude drug cherry leaf extract) | 1.0 |
| Paraben | q.s. |
| Trisodium edetate | 0.05 |
| Colorant | q.s. |
| Perfume | q.s. |
| Purified water | Balance |

| Formulation 8 (cream) | |
|---|---|
| Methylpolysiloxane | 5.0 |
| Decamethylcyclopentasiloxane | 10.0 |
| Polyoxyethylene-methylpolysiloxane copolymer | 0.5 |
| Poly(oxyethylene-oxypropylene)- | |
| methylpolysiloxane copolymer | 0.5 |
| Globular polyalkyl acrylate | 2.0 |
| Glycerol | 5.0 |
| Plant extract (crude drug palo azul) | 0.05 |
| Plant extract (crude drug scutellaria liquid SE) | 2.0 |
| 1,3-Butylene glycol | 5.0 |
| Sodium chloride | 2.0 |
| Sodium metaphosphate | 0.05 |
| Phenoxyethanol | q.s. |
| Trisodium edetate | 0.05 |
| Colorant | q.s. |
| Perfume | q.s. |
| Purified water | Balance |

| Formulation 9 (cream) | |
|---|---|
| Methylpolysiloxane | 5.0 |
| Decamethylcyclopentasiloxane | 15.0 |
| Behenyl alcohol | 1.0 |
| Batyl alcohol | 1.0 |
| Bleached beeswax | 2.0 |
| Glyceryl tri(2-ethylhexanoate) | 2.0 |
| Cetyl octanoate | 2.0 |
| Glycerol | 4.0 |
| Plant extract (crude drug palo azul) | 0.1 |
| Plant extract (crude drug hamamelis) | 0.2 |
| Dipropylene glycol | 6.0 |
| Trisodium edetate | 0.05 |
| Paraben | q.s. |
| Perfume | q.s. |
| Purified water | Balance |

| Formulation 10 (cream) | |
|---|---|
| Vaseline | 1.0 |
| Methylpolysiloxane | 2.0 |
| Squalane | 2.0 |
| Glycerol | 5.0 |
| Dipropylene glycol | 3.0 |
| 1,3-Butylene glycol | 3.0 |
| Polyethylene glycol | 2.0 |
| Plant extract (crude drug palo azul) | 2.0 |
| Plant extract (crude drug scutellaria liquid SE) | 1.0 |
| Potassium hydroxide | 0.25 |
| Sodium metaphosphate | 0.05 |
| Colorant | q.s. |
| Paraben | q.s. |
| Xanthane gum | 0.1 |
| Acrylic acid-alkyl methacrylate copolymer | 0.1 |
| Carboxyvinyl polymer | 0.5 |
| Purified water | Balance |

| Formulation 11 (cream) | |
|---|---|
| Liquid paraffin | 8.0 |
| Vaseline | 2.0 |
| Methylpolysiloxane | 2.0 |
| Stearyl alcohol | 2.0 |
| Behenyl alcohol | 2.0 |
| Pentaerythritol tetraoctanoate | 3.0 |
| Polyoxyethylene glyceryl isostearate | 2.0 |
| Polyoxyethylene glycerol monostearate | 2.0 |
| Lipophilic glycerol monostearate | 2.0 |
| 4-tert-Butyl-4'-methoxybenzoylmethane | 0.05 |
| 2-Ethylhexyl p-methoxycinnamate | 0.5 |
| Hydroxyethyl cellulose | 0.5 |
| Carboxyvinyl polymer | 0.04 |
| Trisodium edetate | 0.05 |
| Plant extract (crude drug palo azul) | 1.0 |
| Plant extract (crude drug lavender oil) | 1.0 |
| Glycerol | 5.0 |
| Dipropylene glycol | 5.0 |
| Perfume | q.s. |
| Paraben | q.s. |
| Purified water | Balance |

| Formulation 12 (cream) | |
|---|---|
| Vaseline | 2.0 |
| Methylpolysiloxane | 2.0 |
| Behenyl alcohol | 0.5 |
| Batyl alcohol | 0.5 |
| Jojoba oil | 3.0 |
| Squalane | 2.0 |
| Pentaerythritol tetraoctanoate | 1.0 |
| Polyoxyethylene hardened castor oil | 0.5 |
| Specially denatured alcohol | 5.0 |
| Plant extract (crude drug palo azul) | 1.5 |
| Plant extract (crude drug pyrola) | 0.5 |
| Glycerol | 5.0 |
| 1,3-Butylene glycol | 5.0 |
| Polyethylene glycol | 2.0 |
| Potassium hydroxide | 0.09 |
| Paraben | q.s. |
| Trisodium edetate | 0.05 |
| Colorant | q.s. |
| Xanthane gum | 0.05 |
| Sodium metaphosphate | 0.05 |
| Carboxyvinyl polymer | 0.25 |
| Purified water | Balance |

| Formulation 13 (cream) | |
|---|---|
| Liquid paraffin | 10.0 |
| Methylpolysiloxane | 5.0 |
| Squalane | 5.0 |
| Pentaerythritol tetra(2-ethylhexanoate) | 5.0 |
| Plant extract (crude drug palo azul) | 0.05 |
| Plant extract (crude drug thymus serphyllum extract) | 2.0 |
| 1,3-Butylene glycol | 5.0 |
| Erythritol | 5.0 |
| Polyethylene glycol | 5.0 |
| Potassium hydroxide | 0.1 |
| Sodium metaphosphate | 0.05 |
| Paraben | q.s. |
| Phenoxyethanol | q.s. |
| Hydroxypropylmethyl cellulose | 0.2 |
| Polyvinyl alcohol | 0.2 |
| Carboxyvinyl polymer | 0.1 |
| Acrylic acid-alkyl methacrylate copolymer | 0.1 |
| Colorant | q.s. |
| Perfume | q.s. |
| Purified water | Balance |

| Formulation 14 (cream) | |
|---|---|
| Liquid paraffin | 5.0 |
| Vaseline | 5.0 |
| Ceresin | 2.0 |
| Squalane | 15.0 |
| Beeswax | 0.5 |
| Polyglyceryl diisostearate | 5.0 |
| Plant extract (crude drug palo azul) | 0.05 |
| Plant extract (crude drug majoram extract) | 1.0 |
| Powdered polyethylene | q.s. |
| Propylene glycol | q.s. |
| Sodium glutamate | q.s. |
| Serine | q.s. |
| Glycine | q.s. |
| Allantoin | q.s. |
| Perfume | q.s. |
| Sodium chloride | q.s. |
| Purified water | Balance |

| Formulation 15 (cream) | |
|---|---|
| Olefin oligomer | 8.0 |
| Vaseline | 5.0 |
| Ceresin | 4.0 |
| Squalane | 18.0 |
| Beeswax | 1.0 |
| Polyglyceryl diisostearate | 5.0 |
| Serine | q.s. |
| Sodium metaphosphate | 0.05 |
| Plant extract (crude drug palo azul) | 0.5 |
| Plant extract (crude drug achillea millefolium) | 1.0 |
| Powdered polyethylene | q.s. |
| Glycerol | 2.0 |
| 1,3-Butylene glycol | 5.0 |
| Sodium chloride | q.s. |
| Powdered polyethylene | 0.1 |
| Paraben | q.s. |
| Perfume | q.s. |
| Colorant | q.s. |
| Purified water | Balance |

| Formulation 16 (cream) | |
|---|---|
| Olefin oligomer | 6.0 |
| Decamethylcyclopentansiloxane | 18.0 |
| Polyoxyethylene-methylpolysiloxane copolymer | 2.0 |
| Methylphenylpolysiloxane | 1.0 |
| Stearyl alcohol | 0.5 |
| Behenyl alcohol | 1.3 |
| Macadamia nut oil fatty acid phytosteryl ester | 0.3 |
| Sodium N-stearoyl-L-glutamate | 0.9 |
| Dimethyldistearylammonium hectorite | 0.8 |
| Glycerol | 4.0 |
| Plant extract (crude drug palo azul) | 5.0 |
| 1,3-Butylene glycol | 5.0 |
| Sodium chloride | 0.05 |
| Paraben | q.s. |
| Phenoxyethanol | q.s. |
| Colorant | q.s. |
| Perfume | q.s. |
| Purified water | Balance |

| Formulation 17 (milky lotion) | |
|---|---|
| Methylpolysiloxane | 2.0 |
| Decamethylcyclopentasiloxane | 2.0 |
| Squalane | 2.0 |
| Specially denatured alcohol | 5.0 |
| Glycerol | 5.0 |
| 1,3-Butylene glycol | 5.0 |
| Polyoxyethylene methylglycoside | 5.0 |
| Plant extract (crude drug palo azul) | 0.02 |
| Potassium hydroxide | 0.1 |
| Sodium metaphosphate | 0.05 |
| Paraben | q.s. |
| Colorant | q.s. |
| Carboxyvinyl polymer | 0.15 |
| Acrylic acid-alkyl methacrylate copolymer | 0.1 |
| Purified water | Balance |

| Formulation 18 (milky lotion) | |
|---|---|
| Methylpolysiloxane | 2.0 |
| Behenyl alcohol | 0.5 |
| Batyl alcohol | 0.5 |
| Hardened oil | 3.0 |
| Squalane | 6.0 |
| Pentaerythritol tetra(2-ethylhexanoate) | 2.0 |
| Polyoxyethylene glyceryl isostearate | 1.5 |
| Polyoxyethylene glycerol monostearate | 1.5 |
| Glycerol | 5.0 |
| 1,3-Butylene glycol | 5.0 |
| Erythritol | 5.0 |
| Plant extract (crude drug palo azul) | 0.05 |
| Potassium hydroxide | 0.05 |
| Sodium metaphosphate | 0.03 |
| Phenoxyethanol | q.s. |
| Carboxyvinyl polymer | 0.12 |
| Purified water | Balance |

| Formulation 19 (milky lotion) | |
|---|---|
| Liquid paraffin | 8.0 |
| Vaseline | 3.0 |
| Decamethylcyclopentasiloxane | 1.0 |
| Behenyl alcohol | 2.0 |
| Jojoba oil | 1.0 |
| Stearic acid | 1.0 |
| Behenic acid | 0.5 |
| Pentaerythritol tetra(2-ethylhexanoate) | 3.0 |
| Cetyl 2-ethylhexanoate | 3.0 |
| Self-emulsifiable glycerol monostearate | 1.5 |
| 2-Ethylhexyl p-methoxycinnamate | 0.1 |
| Glycerol | 8.0 |
| Plant extract (crude drug palo azul) | 5.0 |
| Dipropylene glycol | 5.0 |
| Polyethylene glycol | 5.0 |
| Potassium hydroxide | 0.1 |
| Sodium metaphosphate | 0.05 |
| Trisodium edetate | 0.05 |
| Paraben | q.s. |
| Colorant | q.s. |
| Perfume | q.s. |
| Purified water | Balance |

| Formulation 20 (milky lotion) | |
|---|---|
| Decamethylcyclopentasiloxane | 15.0 |
| Polyoxyethylene-methylpolysiloxane polymer | 3.0 |
| Macadamia nut oil | 1.0 |
| Squalane | 2.0 |
| Cholesteryl hydroxystearate | 0.2 |
| Cetyl 2-ethylhexanoate | 2.0 |
| Distearyldimethylammonium chloride | 0.3 |
| Glycerol | 8.0 |
| 1,3-Butylene glycol | 5.0 |
| Plant extract (crude drug palo azul) | 3.0 |
| Maltitol solution | 2.0 |
| Trisodium edetate | 0.05 |
| Aluminum magnesium silicate | 0.3 |
| Paraben | q.s. |
| Colorant | q.s. |
| Ion-exchanged water | Balance |

| Formulation 21 (milky lotion) | |
|---|---|
| Squalane | 0.2 |
| Cholesteryl hydroxystearate | 0.1 |
| Glycerol 2-ethylhexanoate | 0.4 |
| Polyoxyethylene-hardened castor oil | 0.4 |
| Polyglyceryl diisostearate | 0.3 |
| Denatured alcohol | 5.0 |
| Glycerol | 2.0 |
| Plant extract (crude drug palo azul) | 1.0 |
| Dipropylene glycol | 5.0 |
| Polyethylene glycol | 2.0 |
| Potassium hydroxide | 0.6 |
| Trisodium edetate | 0.05 |
| Xanthane gum | 0.2 |
| Hydroxyethyl cellulose | 0.03 |
| Carboxyvinyl polymer | 0.3 |
| Perfume | q.s. |
| Paraben | q.s. |
| Purified water | Balance |

| Formulation 22 (milky lotion) | |
|---|---|
| Vaseline | 5.0 |
| Behenyl alcohol | 0.5 |
| Hardened oil | 1.5 |
| Jojoba oil | 2.5 |
| Squalane | 4.0 |
| Pentaerythritol tetraoctanoate | 3.0 |
| Polyoxyethylene-hardened castor oil | 0.5 |
| Glycerol | 5.0 |
| 1,3-Butylene glycol | 5.0 |
| Polyethylene glycol | 2.0 |
| Plant extract (crude drug palo azul) | 1.0 |
| Potassium hydroxide | 0.07 |
| Carboxyvinyl polymer | 0.2 |
| Paraben | q.s. |
| Colorant | q.s. |
| Purified water | Balance |

| Formulation 23 (milky lotion) | |
|---|---|
| Vaseline | 2.0 |
| Methylpolysiloxane | 2.0 |
| Behenyl alcohol | 0.3 |
| Batyl alcohol | 0.3 |
| Cholesteryl hydroxystearate | 0.1 |
| 4-tert-Butyl-4'-methoxybenzoylmethane | 0.1 |
| Denatured alcohol | 3.0 |
| Glycerol | 8.0 |
| Plant extract (crude drug palo azul) | 1.0 |
| 1,3-Butylene glycol | 4.0 |
| Polyethylene glycol | 3.0 |
| Coconut oil fatty acid-hydrolyzed collagen | |
| potassium solution | 0.2 |
| Potassium hydroxide | 0.08 |
| Trisodium edetate | 0.05 |
| Xanthane gum | 0.05 |
| Carboxyvinyl polymer | 0.22 |
| Colorant | q.s. |
| Paraben | q.s. |
| Perfume | q.s. |
| Purified water | Balance |

| Formulation 24 (milky lotion) | |
|---|---|
| Vaseline | 0.7 |
| Methylpolysiloxane | 0.3 |
| Behenyl alcohol | 0.7 |
| Squalane | 2.0 |
| Cetyl 2-ethylhexanoate | 0.5 |
| Sodium N-stearoyl-L-glutamate | 0.3 |
| Denatured alcohol | 3.0 |
| Glycerol | 4.0 |
| Dipropylene glycol | 6.0 |
| 1,3-Butylene glycol | 4.0 |
| Plant extract (crude drug palo azul) | 1.0 |
| Potassium hydroxide | 0.07 |
| Sodium metaphosphate | 0.01 |
| Xanthane gum | 0.02 |
| Carboxyvinyl polymer | 0.14 |
| Colorant | q.s. |
| Paraben | q.s. |
| Perfume | q.s. |
| Purified water | Balance |

| Formulation 25 (toilet water) | |
|---|---|
| 1,3-Butylene glycol | 6.0 |
| Glycerol | 4.0 |
| Oleyl alcohol | 0.1 |
| POE(20) sorbitan monolaurate | 0.5 |
| POE(15) lauryl alcohol ether | 0.5 |
| Plant extract (crude drug palo azul) | 1.0 |
| Plant extract (crude drug hamamelis) | 0.5 |
| Ethanol | 10.0 |
| Perfume | q.s. |
| Colorant | q.s. |
| Purified water | Balance |

| Formulation 26 (toilet water) | |
|---|---|
| Sorbitol | 4.0 |
| Dipropylene glycol | 6.0 |
| PEG 1500 | 5.0 |
| POE(20) oleyl alcohol ether | 0.5 |
| Methyl cellulose | 0.2 |
| Pyrus cydonia seed extract | 0.1 |
| Plant extract (crude drug palo azul) | 0.5 |
| Plant extract (crude drug scutellaria liquid SE) | 0.1 |
| Ethanol | 10.0 |
| Perfume | q.s. |
| Colorant | q.s. |
| Purified water | Balance |

| Formulation 27 (toilet water) | |
|---|---|
| 1,3-Butylene glycol | 6.0 |
| Glycerol | 3.0 |
| PEG 4000 | 3.0 |
| Olive oil | 0.5 |
| POE(20) sorbitan monolaurate | 1.5 |
| POE(5) oleyl alcohol ether | 0.3 |
| Ethanol | 10.0 |
| Plant extract (crude drug palo azul) | 0.3 |
| Plant extract (crude drug majoram extract) | 1.5 |
| Perfume | q.s. |
| Colorant | q.s. |
| Purified water | Balance |

| Formulation 28 (toilet water) | |
|---|---|
| Dipropylene glycol | 1.0 |
| Sorbitol | 1.0 |
| POE(20) oleyl alcohol ether | 1.0 |
| Zinc sulfocarbolate | 0.2 |
| Citric acid | 0.1 |
| Ethanol | 15.0 |
| Plant extract (crude drug palo azul) | 0.3 |
| Plant extract (crude drug rosa roxburghii fruit) | 0.1 |
| Perfume | q.s. |
| Colorant | q.s. |
| Purified water | Balance |

| Formulation 29 (toilet water) | |
|---|---|
| 1,3-Butylene glycol | 6.0 |
| Dipropylene glycol | 6.0 |
| PEG 400 | 6.0 |
| POE(20) sorbitan monolaurate | 1.0 |
| Polyoxyethylene-polyoxypropylene block polymer | 1.5 |
| Ethanol | 15.0 |
| Plant extract (crude drug scutellaria liquid SE) | 0.3 |
| Plant extract (crude drug rosa roxburghii fruit) | 0.1 |
| Perfume | q.s. |
| Colorant | q.s. |
| Purified water | Balance |

| Formulation 30 (cream) | |
|---|---|
| Behenyl alcohol | 1.0 |
| Stearyl alcohol | 2.0 |
| Squalane | 10.0 |
| Pentaerythrityl tetraoctanoate | 5.0 |
| Lanolin | 5.0 |
| Paraben | 0.3 |
| Polyoxyethylene behenyl alcohol | 1.0 |
| Glycerol | 10.0 |
| Hypotaurine | 0.1 |
| Sodium acetylhyaluronate | 0.1 |
| Purified water | Balance |

| Formulation 31 (milky lotion) | |
|---|---|
| Squalane | 10.0 |
| Isopropyl myristate | 20.0 |
| Decamethylcyclopentanehexane | 35.0 |
| Alkyl-modified carboxyvinyl polymer | 0.05 |
| Sorbitan monooleate | 5.0 |
| Dipropylene glycol | 3.0 |
| Glutathione | 1.0 |
| Purified water | Balance |

| Formulation 32 (milky lotion) | |
|---|---|
| Liquid paraffin | 10.0 |
| Octamethylcyclotetrasiloxane | 5.0 |
| 1,3-Butyl glycol | 10.0 |
| Glycerol | 3.0 |
| Ascorbic acid derivative | 3.0 |
| Glutathione | 0.1 |
| Carboxyvinyl polymer | 0.15 |
| Xanthane gum | 0.25 |
| Stearic acid | 1.5 |
| Cetyl alcohol | 0.5 |
| Polyoxyethylene(10) monooleate | 1.0 |
| Stearic acid monoglyceride | 1.0 |
| Ethanol | 3.0 |
| Perfume | q.s. |
| Purified water | Balance |

| Formulation 33 (cream) | |
|---|---|
| Cetostearyl alcohol | 3.5 |
| Squalane | 20.0 |
| Beeswax | 3.0 |
| Lanolin | 5.0 |
| Paraben | 0.3 |
| Polyoxyethylene(20) sorbitan monopalmitate | 2.0 |
| Stearic acid monoglyceride | 2.0 |
| Hypotaurine | 0.1 |
| Vitamin A | 2.0 |
| Perfume | q.s. |
| Purified water | Balance |

| Formulation 34 (foundation) | |
|---|---|
| Talc | 3.0 |
| Titanium dioxide | 5.0 |
| Red iron oxide | 0.5 |
| Yellow iron oxide | 1.4 |
| Black iron oxide | 1.0 |
| Polyoxyethylene sorbitan monostearate | 0.9 |
| Triethanolamine | 1.0 |
| Propylene glycol | 5.0 |
| Hypotaurine | 1.0 |
| Stearic acid | 2.2 |
| Isohexadecyl alcohol | 7.0 |
| Stearic acid monoglyceride | 2.0 |
| Lanolin | 2.0 |
| Paraffin | 8.0 |
| Paraben | q.s. |
| Perfume | q.s. |

| Formulation 35 (cream) | |
|---|---|
| Liquid paraffin | 3 |
| Vaseline | 1 |
| Methylpolysiloxane | 1 |
| Stearyl alcohol | 1.8 |
| Behenyl alcohol | 1.6 |
| Glycerol | 8 |
| Dipropylene glycol | 5 |
| Macadamia nut oil | 2 |
| Hardened oil | 3 |
| Squalane | 6 |
| Xylitol | 3 |
| Stearic acid | 2 |
| Cholesteryl hydroxystearate | 0.5 |
| Cetyl 2-ethylhexanoate | 4 |
| Self-emulsifiable glycerol monostearate | 3 |
| Potassium hydroxide | 0.15 |
| Sodium metaphosphate | 0.05 |
| Trimethylglycine | 2 |
| DL-α-Tocopherol 2-L-ascorbic acid diphosphate potassium | 1 |
| DL-α-Tocopherol acetate | 0.1 |
| Palo azul extract (crude drug palo azul) | 0.05 |
| Rubus suavissimus extract | 0.1 |
| Paraben | q.s. |
| Trisodium edetate | 0.05 |
| 4-t-Butyl-4'-methoxydibenzoylmethane | 0.05 |
| Glyceryl di-p-methoxycinnamate | |
| mono-2-ethylhexanoate | 0.05 |
| Red iron oxide | q.s. |
| Yellow iron oxide | q.s. |
| Carboxyvinyl polymer | 0.05 |
| Purified water | Balance |

| Formulation 36 (cream) | |
|---|---|
| Liquid paraffin | 8 |
| Vaseline | 3 |
| Methylpolysiloxane | 2 |
| Stearyl alcohol | 3 |
| Behenyl alcohol | 2 |
| Glycerol | 5 |
| Dipropylene glycol | 4 |
| Xylitol | 1 |
| Pentaerythritol tetra(2-ethylhexanoate) | 4 |
| Polyoxyethylene glyceryl isostearate | 2 |
| Polyoxyethylene glycerol monostearate | 1 |
| Lipophilic glycerol monostearate | 2 |
| Citric acid | 0.05 |
| Sodium citrate | 0.05 |
| Potassium hydroxide | 0.015 |
| Palo azul extract (crude drug palo azul) | 0.1 |
| Glycyrrhiza flavonoid | 0.1 |
| Retinol palmitate (1,000,000 units) | 0.25 |
| DL-α-Tocopherol acetate | 0.1 |
| Paraben | q.s. |
| Phenoxyethanol | q.s. |
| Dibutylhydroxytoluene | 0.1 |
| Trisodium edetate | 0.05 |
| 4-t-Butyl-4'-methoxydibenzoylmethane | 0.01 |
| 2-ethylhexyl p-methoxycinnamate | 0.1 |
| β-Carotene | 0.01 |
| Polyvinyl alcohol | 0.5 |
| Hydroxyethyl cellulose | 0.5 |
| Carboxyvinyl polymer | 0.05 |
| Purified water | Balance |
| Perfume | q.s. |

| Formulation 37 (moisture cream) | |
|---|---|
| Liquid paraffin | 10 |
| Methylpolysiloxane | 2 |
| Glycerol | 10 |
| 1,3-Butylene glycol | 2 |
| Erythritol | 1 |
| Xylitol | 1.5 |
| Polyethylene glycol 1500 | 5 |
| Squalane | 15 |
| Pentaerythritol tetra(2-ethylhexanoate) | 5 |
| Potassium hydroxide | 0.1 |
| Palo azul extract (crude drug palo azul) | 0.08 |
| Sodium metaphosphate | 0.05 |
| Tocopherol acetate | 0.05 |
| Paraben | q.s. |
| Hydroxypropyl methylcellulose | 0.3 |
| Polyvinyl alcohol | 0.1 |
| Carboxyvinyl polymer | 0.2 |
| Acrylic acid-alkyl methacrylate copolymer | 0.1 |
| Purified water | Balance |

| Formulation 38 (cream (oil-free)) | |
|---|---|
| Cyclomethicon | 30 |
| Polyoxyethylene-methylpolysiloxane copolymer | 1.5 |
| Glycerol | 5 |
| Dipropylene glycol | 5 |
| Xylitol | 3 |
| Talc | 5 |
| Paraben | q.s. |
| Palo azul extract (crude drug palo azul) | 0.03 |
| Phenoxyethanol | 0.3 |
| Trisodium edetate | 0.02 |
| Dimethyldistearylammonium hectorite | 0.5 |
| Purified water | Balance |
| Trimethylsiloxysilicic acid | 0.5 |
| Globular silicic acid anhydride | 0.5 |
| Finely divided titanium oxide (hydrophobized | |
| product, 30 nm) | 7 |
| Globular polyethylene powder | 2 |
| poly(oxyethylene-oxypropylene)-methylpolysiloxane copolymer | 1 |

| Formulation 39 (milky lotion) | |
|---|---|
| Liquid paraffin | 7 |
| Vaseline | 3 |
| Decamethylcyclopentasiloxane | 2 |
| Behenyl alcohol | 2 |
| Glycerol | 5 |
| Dipropylene glycol | 7 |
| Polyethylene glycol 1500 | 2 |
| Xylitol | 2 |
| Jojoba oil | 1 |
| Isostearic acid | 0.5 |
| Stearic acid | 0.5 |
| Behenic acid | 0.5 |
| Pentaerythritol tetra(2-ethylhexanoate) | 3 |
| Cetyl 2-ethylhexanoate | 3 |
| Glycerol monostearate | 1 |
| Polyoxyethylene glycerol monostearate | 1 |
| Potassium hydroxide | 0.1 |
| Sodium hexametaphosphate | 0.05 |
| Stearyl glycyrrhetinate | 0.05 |
| L-Arginine | 0.1 |
| Royal jelly extract | 0.1 |
| Tocopherol acetate | 0.1 |
| Sodium hyaluronate | 0.1 |
| Palo azul extract (crude drug palo azul) | 0.05 |
| Paraben | q.s. |
| Trisodium edetate | 0.05 |
| 4-t-Butyl-4'-methoxydibenzoylmethane | 0.1 |
| 2-Ethylhexyl p-methoxycinnamate | 0.1 |
| Yellow iron oxide | q.s. |
| Carboxyvinyl polymer | 0.15 |
| Purified water | Balance |
| Perfume | q.s. |

| Formulation 40 (milky lotion) | |
|---|---|
| Methylpolysiloxane | 2 |
| Behenyl alcohol | 1 |
| Batyl alcohol | 0.5 |
| Xylitol | 2 |
| Erythritol | 2 |
| Hardened oil | 3 |
| Squalane | 6 |
| Pentaerythritol tetra(2-ethylhexanoate) | 2 |
| Polyoxyethylene glyceryl isostearate | 1 |
| Polyoxyethylene glycerol monostearate | 1 |
| Palo azul extract (crude drug palo azul) | 0.1 |
| Potassium hydroxide | q.s. |
| Sodium hexametaphosphate | 0.05 |
| Phenoxyethanol | q.s. |
| Carboxyvinyl polymer | 0.11 |
| Purified water | Balance |

| Formulation 41 (milky lotion for wrinkle care) | |
|---|---|
| Decamethylcyclopentasiloxane | 150 |
| Trimethylsiloxysilicic acid | 5 |
| Polyoxyethylene-methylpolysiloxane copolymer | 5 |
| Glycerol | 5 |
| 1,3-Butylene glycol | 5 |
| Maltitol solution | 2 |
| Macadamia nut oil | 2 |
| Squalane | 2 |
| Xylitol | 3 |
| Cholesteryl hydroxystearate | 0.5 |
| Cetyl 2-ethylhexanoate | 2 |
| Distearyldimethylammonium chloride | 0.2 |
| L-Ascorbic acid sulfate disodium | 0.1 |
| DL-α-Tocopherol 2-L-ascorbic acid diphosphate | |
| potassium | 0.1 |
| Tocopherol acetate | 0.05 |
| Fish collagen | 0.4 |
| Chondroitin sulfate sodium | 0.01 |
| Sodium hyaluronate | 0.1 |
| Palo azul extract (crude drug palo azul) | 0.1 |
| Paraben | q.s. |
| Trisodium edetate | 0.05 |
| Glyceryl di-p-methoxycinnamate | |
| mono-2-ethylhexanoate | 0.05 |
| Aluminum magnesium silicate | 0.3 |
| Purified water | Balance |

| Formulation 42 (milky lotion) | |
|---|---|
| Methylpolysiloxane | 3 |
| Decamethylcyclopentasiloxane | 4 |
| Ethyl alcohol | 5 |
| Glycerol | 6 |
| 1, 3-Butylene glycol | 5 |
| Polyethylene methylglucoside | 3 |
| Sunflower oil | 1 |
| Squalane | 2 |
| Xylitol | 3 |
| Potassium hydroxide | 0.1 |
| Sodium hexametaphosphate | 0.05 |
| Hydroxypropyl-β-cyclodextrin | 0.1 |
| Disodium glycyrrhizinate | 0.05 |
| Eriobotrya japonica leaf extract | 0.1 |
| Sodium L-glutamate | 0.05 |
| Fennel extract | 0.1 |
| Lavender oil | 0.1 |
| Palo azul extract (crude drug palo azul) | 0.05 |
| Paraben | q.s. |
| Dimorpholinopyridazine | 0.1 |
| Red iron oxide | q.s. |
| Yellow iron oxide | q.s. |
| Xanthane gum | 0.1 |
| Carboxyvinyl polymer | 0.1 |
| Acrylic acid-alkyl methacrylate copolymer | 0.1 |
| Purified water | Balance |

| Formulation 43 (milky lotion) | |
|---|---|
| Vaseline | 5 |
| Behenyl alcohol | 0.4 |
| Batyl alcohol | 0.4 |
| Glycerol | 7 |
| 1,3-Butylene glycol | 7 |
| Xylitol | 3 |
| Polyethylene glycol 20000 | 2 |
| Hardened oil | 2 |
| Jojoba oil | 2 |
| Squalane | 5 |
| Pentaerythritol tetra(2-ethylhexanoate) | 2 |
| Polyoxyethylene-hardened castor oil | 0.5 |
| Betaine lauryl dimethylaminoacetate | 0.4 |
| Potassium hydroxide | q.s. |
| Sodium pyrosulfite | 0.01 |
| Sodium hexametaphosphate | 0.05 |
| Disodium glycyrrhizinate | 0.05 |
| Trimethylglycine | 3 |
| Arbutin | 3 |
| Yeast extract | 0.1 |
| Tocopherol acetate | 0.1 |
| Thiotaurine | 0.1 |
| Palo azul extract (crude drug palo azul) | 0.05 |
| Sophora augustifolia extract | 0.1 |
| Phenoxyethanol | q.s. |
| Red iron oxide | q.s. |
| Pyrus cydonia seed extract | 0.1 |
| Carboxyvinyl polymer | 0.2 |
| Purified water | Balance |

| Formulation 44 (toilet water) | |
|---|---|
| Ethyl alcohol | 5 |
| Glycerol | 2 |
| 1,3-Butylene glycol | 5 |
| Polyoxyethylene polyoxypropylene decyl | |
| tetradecyl ether | 0.2 |
| Sodium hexametaphosphate | 0.03 |
| Trimethylglycine | 1 |
| Xylitol | 3 |
| Sodium polyaspartate | 0.1 |
| DL-α-Tocopherol 2-L-ascorbic acid diphosphate | |
| potassium | 0.1 |
| Thiotaurine | 0.1 |
| Green tea extract | 0.1 |
| Palo azul extract (crude drug palo azul) | 0.05 |
| Iris extract | 0.1 |
| Phenoxyethanol | 0.12 |
| Ethylenediamine hydroxyethylacetic acid trisodium | |
| (HEDTA) | 0.07 |
| Carboxyvinyl polymer potassium | 0.09 |
| Purified water | Balance |
| Perfume | q.s. |

| Formulation 45 (toilet water) | |
|---|---|
| Glycerol | 2 |
| 1,3-Butylene glycol | 4 |
| Erythritol | 2 |
| Xylitol | 3 |
| Palo azul extract (crude drug palo azul) | 0.2 |
| Polyoxyethylene methylglucoside | 1 |
| Polyoxyethylene-hardened castor oil | 0.5 |
| Citric acid | 0.02 |
| Sodium citrate | 0.08 |
| Phenoxyethanol | 0.25 |
| N-Coconut oil fatty acid acyl L-arginine ethyl DL-pyrrolidonecarboxylate | 0.1 |
| Purified water | Balance |

| Formulation 46 (toilet water) | |
|---|---|
| Ethyl alcohol | 5 |
| Glycerol | 0.1 |
| Dipropylene glycol | 2 |
| 1,3-Butylene glycol | 6 |
| Xylitol | 6 |
| Rosemary oil | 0.001 |
| Sage oil | 0.001 |
| Citric acid | 0.02 |
| Sodium citrate | 0.08 |
| Sodium hexametaphosphate | 0.03 |
| Hydroxypropyl-β-cyclodextrin | 0.1 |
| Soapberry extract | 0.1 |
| Rose fruit extract | 0.1 |
| Lily extract | 0.1 |
| Phellodendron extract | 0.1 |
| Rosa wichuraiana extract | 0.1 |
| Raspberry extract | 0.1 |
| Lavender oil | 0.1 |
| Peach kernel extract | 0.1 |
| Palo azul extract (crude drug palo azul) | 0.05 |
| Sodium alginate | 0.001 |
| Purified water | Balance |

### Industrial Applicability

The external preparations of the present invention can significantly prevent human keratinocytes from undergoing a reduction in the moisture content of the stratum corneum as a result of drying and can suppress a reduction in the skin barrier function of the stratum corneum. Accordingly, the present invention may be utilized in the industry for the manufacture of cosmetics or dermatological preparations.

## Claims

1. An external preparation for the skin comprising a substance capable of inhibiting significantly a reduction in the expression level of the filaggrin gene in cultured human keratinocytes as induced by exposure to a gaseous phase, in a sufficient amount to inhibit a reduction in the barrier function of the stratum corneum in the human skin and a reduction in the moisture-retaining function thereof and a base or additive acceptable for use in cosmetics and/or dermatological preparations.

2. An external preparation for the skin as claimed in Claim 1 wherein the substance capable of inhibiting significantly a reduction in the expression level of the filaggrin gene comprises one or more members selected from the group consisting of betaine compounds and derivatives thereof, polyols and derivatives thereof, crude drugs, and substances having an antioxidant action.

3. An external preparation for the skin as claimed in Claim 2 wherein the betaine compounds and derivatives thereof are selected from the group consisting of glycinebetaine, sultaine, γ-butyrobetaine, decylbetaine, laurylbetaine, myristylbetaine, cetylbetaine, stearylbetaine, behenylbetaine, lauramidopropylbetaine, oleamidopropylbetaine, palmitamidopropylbetaine, γ-butyrobetaine, laurylsultaine, coco-sultaine, poly(methacryloyloxyethylbetaine) and poly(methacryloyloxyethylbetaine-co-2-hydroxyethylmethacrylic acid).

4. An external preparation for the skin as claimed in Claim 2 wherein the polyols and derivatives thereof are selected from the group consisting of glycerol, 1,3-propanediol, 2-methyl- 1, 3-propanediol, 1,4-butanediol, 1,5-pentanediol, diglycerol, erythritol, gluconic acid, 1,2,6-hexanetriol, inositol, lactitol, maltitol, mannitol and xylitol.

5. An external preparation for the skin as claimed in Claim 2 wherein the crude drugs are selected from the group consisting of crude drug carqueja (South America) derived from a plant of the genus Baccharis, particularly Baccharis genistelloides, crude drug macelamista (South America) derived from a plant of the genus Achyrocline, particularly Achyrocline satureoides, and crude drug achillea millefolium derived from a plant of the genus Achillea, particularly Achillea millefolium L., these genera belonging to the family Compositae; crude drug thymus serphyllum extract derived from a plant of the genus Thymus, particularly Thymus serphyllum Linne, subsp. serphyllum, crude drug majoram extract derived from a plant of the genus Origanum, particularly Origanum majorana L., crude drug scuttellaria root derived from a plant of the genus Scuttellaria, particularly Scuttellaria baicalensis Georgi, and crude drug lavender oil derived from a plant of the genus Lavandula, particularly Lavandula officinalls. these genera belonging to the family Labiatae; crude drug rosa roxburghii fruit derived from a plant of the genus Rosa, particularly Rosa roxburghii, and crude drug cherry leaf extract derived from a plant of the genus Prunus, particularly Prunus lannesiana (Corr.) Wilson var. Spciosa (Koidz) Makino, these genera belonging to the family Rosaceae; crude drug hamamelis derived from a plant of the genus Hamamelis of the family Hamamelidaceae, particularly Hamamelis virginiana L.; crude drug pyrola derived from a plant of the genus Pyrola of the family Pyrolaceae. particularly Pyrola japonicus Klenze; and crude drug palo azul (South America) derived from a plant of the genus Eysenhardtia of the family Leguminosae, particularly Evsenhardtia polistachya.

6. An external preparation for the skin as claimed in Claim 2 wherein the substances having an antioxidant action are selected from the group consisting of ascorbic acid and salts or derivatives thereof, and sulfur-containing amino acid containing compounds and intermediary metabolites thereof.

7. An external preparation for the skin as claimed in Claim 2 wherein the substances having an antioxidant action are selected from the group consisting of glutathione and hypotaurine.

8. An external preparation for the skin as claimed in Claim 2 which contains a combination of a crude and one or more members selected from the group consisting of betaine compounds and derivatives thereof, polyols and derivatives thereof, and substances having an antioxidant action.

9. An external preparation for the skin as claimed in Claim 2 which contains crude drug palo azul as a crude drug and either of glycerol and a sugar alcohol having 4 to 6 carbon atoms ; crude drug palo azul as a crude drug, glycerol, and glutathione or hypotaurine ; crude drug palo azul as a crude drug, glycerol, and ascorbic acid or its salt or derivative; or crude drug palo azul as a crude drug, glycerol, and a betaine compound or its derivative.

10. An external preparation for the skin as claimed in Claim 2 which contains crude drug palo azul as a crude drug, glycerol and xylitol.

11. An external preparation for the skin as claimed in Claim 2 which contains either of glycerol and a sugar alcohol having 4 to 6 carbon atoms, and a crude drug.

12. An external preparation for the skin containing crude drug palo azul as a crude drug and either of glycerol and a sugar alcohol having 4 to 6 carbon atoms; crude drug palo azul as a crude drug, glycerol, and glutathione or hypotaurine; crude drug palo azul as a crude drug, glycerol, and ascorbic acid or its salt or derivative; or crude drug palo azul as a crude drug, glycerol, and a betaine compound or its derivative.

13. An external preparation for the skin as claimed in Claim 12 which contains crude drug palo azul as a crude drug, glycerol and xylitol.

14. The use of a substance capable of inhibiting significantly a reduction in the expression level of the filaggrin gene in cultured human keratinocytes as induced by exposure to a gaseous phase, for the making of an external preparation for the skin which can inhibit a reduction in the barrier function of the stratum corneum in the human skin.

15. The use as claimed in Claim 14 wherein the substance capable of inhibiting significantly a reduction in the expression level of the filaggrin gene comprises one or more members selected from the group consisting of betaine compounds and derivatives thereof, polyols and derivatives thereof, crude drugs, and substances having an antioxidant action.

16. The use as claimed in Claim 14 or 15 wherein the substance capable of inhibiting significantly a reduction in the expression level of the filaggrin gene comprises palo azul as a crude drug and either of glycerol and a sugar alcohol having 4 to 6 carbon atoms; palo azul as a crude drug, glycerol, and glutathione or hypotaurine; palo azul as a crude drug, glycerol, and ascorbic acid or its salt or derivative; or crude drug palo azul as a crude drug, glycerol, and a betaine compound or its derivative.

17. A method for inhibiting or restoring skin damage caused by drying which comprises the step of applying to the human skin a composition containing one or more members selected from the group consisting of betaine compounds and derivatives thereof, polyols and derivatives thereof, crude drugs, and substances having an antioxidant action.

18. A method as claimed in claim 17 wherein the composition contains palo azul as a crude drug and either of glycerol and a sugar alcohol having 4 to 6 carbon atoms; palo azul as a crude drug, glycerol, and glutathione or hypotaurine; crude drug palo azul as a crude drug, glycerol, and ascorbic acid; or crude drug palo azul as a crude drug, glycerol, and a betaine compound or its derivative.

19. A method for evaluating whether a substance can inhibit skin damage caused by drying or not, the method comprising
(A) providing cultured human keratinocytes,
(B) after the cultured human keratinocytes are exposed to a gaseous phase in the presence of a test substance, detecting the expression level of the filaggrin gene in the keratinocytes, and
(C) comparing the detected expression level of the filaggrin gene with that of a control, and regarding the level of the expression as an index to the skin damage-inhibiting effect.

20. An evaluation method as claimed in Claim 19 wherein the expression level of the filaggrin gene is determined by measuring the amount of filaggrin protein in the human keratinocytes or the amount of mRNA for filaggrin.

21. An evaluation method as claimed in Claim 19 wherein the cultured human keratinocytes are obtained by culturing normal cells of human preputial origin on a feeder layer.

22. An evaluation method as claimed in Claim 19 wherein, when a reduction in the expression level of the filaggrin gene in the keratinocytes having been exposed to a gaseous phase is inhibited in the presence of a test substance as compared with that observed in the absence of the test substance (control), the test substance is evaluated to be capable of inhibiting skin damage caused by drying.

23. An evaluation method as claimed in Claim 19 wherein the exposure to a gaseous phase is carried out by removing the culture medium of the cultured human keratinocytes.
